# EUROPEAN PATENT APPLICATION

(11) **EP 2 163 558 A2**
(43) Date of publication of application: **17.03.2010**
(21) Application number: 09178401.7
(22) Date of filing: 04.10.2005
(51) Int. Cl.: C07K 1/20, C07K 7/06, C07K 14/655

(54) **A Counterion Exchange Process for Peptides**

(30) Priority: 04.10.2004 US 616010 P; 22.11.2004 US 630528 P
(62) Divisional of application: 05811089.1
(71) Applicant: Novetide, Ltd., 26111 Haifa Bay (IL)
(72) Inventor: Tovi, Avi, 20104, Zurit (IL); Eidelman, Chaim, 25167, Oshrat (IL); Elster, Shai, 32811, Haifa (IL); Alon, Hagi, 25125, Kibutz Yehiam (IL); Ivchenko, Alexander, 28076, Keryat Ata (IL); Butilca, Gabriel-Marcus, 20100, Karmiel (IL); Zaoui, Gil, 27070, Keryat Bialik (IL); Shusman, Shimon, 21926, Karmiel (IL)
(74) Representative: Lumsden, Stuart Edward Henry

(57) **Abstract**

The invention encompasses a process for purifying a peptide comprising loading a peptide onto a RP-HPLC column; washing the column with an aqueous solution of a pharmaceutically acceptable counterion salt; and eluting the peptide from the column with a solvent mixture of a organic solvent and an acid of the pharmaceutically acceptable counterion, wherein the aqueous solution has a pH of at least about 6.

## Description

### Cross-reference to Related Applications

This application claims the benefit of provisional applications Serial Nos. 60/616,010, filed October 4, 2004; and 60/630,528, filed November 22, 2004, which are incorporated herein by reference.

### Field of the Invention

The invention encompasses the purification of peptides using a counterion exchange process.

### Background of the Invention

Somatostatin is known to possess a very broad therapeutic potential and can be administered in a wide variety of clinical applications. The mean half-life in plasma of somatostatin is extremely short, therefore reducing the potential number of possible applications of this polypeptide. Research was carried out with the aim of developing analogs of somatostatin which exhibited greater stability and efficacy. One series of compounds which were evaluated as potentially useful somatostatin analogs were cyclic octapeptides. Evaluation of the cyclic octapeptide, octreotide, demonstrated that the compound had excellent biological activity both in vitro and in vivo (Pless J., Metabolism 41, 5-6 (1992)). Octreotide has the following basic formula: 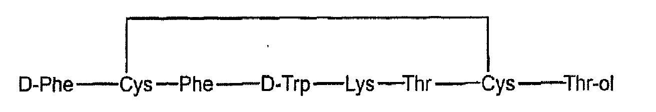 (SEQ. ID. NO. 1) wherein the sulfur atoms of the Cys residues at the positions 2 and 7 are bounded by a disulfide bridge. The carboxylic group of the C-terminal amino acid, threonine (Thr), is reduced to the alcohol Thr-ol (threoninol) residue.

The presence of D-phenylalanine (D-Phe) at the N-terminal end and of an amino alcohol at the C-terminal end, along with the D-tryptophan (D-Trp) residue and the disulfide bridge, make the molecule very resistant to metabolic degradation. Octreotide permits a 24 hour incubation in aggressive mediums, such as gastric juices or in intestinal mucosa.

Octreotide inhibits growth hormone for a lengthy period, inhibits the secretion of glucagon to a lesser degree, and inhibits insulin secretion only in a transient manner. Thus, octreotide is selective more than other somatostatin analogues in regulating the levels of growth hormone in the body and therefore, presently is indicated in acromegaly to control and reduce the plasma levels of such hormone. Also, octreotide is useful in the treatment of cellular alterations of gastroenteropancreatic endocrine origin and of certain types of tumors.

The synthesis of octreotide and its derivatives has been described by two general synthetic methods. The first method is a solution phase procedure, based on fragment condensation, as described by Bauer *et al.* European Patent Application No. 29,579 (1981) and U.S. Patent No. 4,395,403. The process generally comprises removing a protecting group from a protected hexapeptide residue; linking together two peptide units by an amide bond, wherein one comprises a hexapeptide residue; converting a functional group at the N- or C-terminal end of the resulting polypeptide; and oxidizing the polypeptide. The process involves a time-consuming, multi-step synthesis, and presents additional problems during the separation of octreotide from the reaction mixtures because all the synthetic steps are carried out in solution phase.

The second method for the synthesis of octreotide synthesizes the entire peptide chain using solid phase peptide synthesis, starting the synthesis at the threoninol residue. This method requires that the threoninol residue be protected.

The second synthetic process uses an aminomethyl resin upon which the threoninol residue is incorporated with the two alcohol functions protected in acetal form. Mergler et al., "Peptides: Chemistry and Biology," Proceedings of the 12th American Peptide Symposium, Poster 292 Presentation (Smith, J. A. and Rivier J. E., Eds ESCOM, Leiden) (1991). The synthesis is carried out following an Fmoc/t-Bu protection scheme; forming the disulfide bridge on a resin by oxidation of the thiol groups of the previously deprotected cysteine residues; and releasing and deprotecting the peptide with a 20% mixture of TFA/DCM.

Alsina *et al.* described the incorporation of a threoninol residue on active carbonate resins wherein the amino group is protected by a Boc group and the side chain is protected by a Bzl group. Alsina et al., Tetrahedron Letters, 38, 883-886 (1997). Thereafter, the synthesis continued using a Boc/Bzl strategy. Formation of the disulfide bridge was carried out directly on resin using iodine, and the peptide was cleaved from the resin and its side chain protecting groups were simultaneously removed with HF/anisole (9/1). At a final stage the formyl group was removed with a piperidine/DMF solution. Neugebauer *et al*. described a linear synthesis with a yield of only 7%. Neugebauer et al., PEPTIDES: CHEMISTRY, STRUCTURE AND BIOLOGY, p. 1017 (Marshal G. R. and Rivier J. E., Eds ESCOM, Leiden, 1990).

Edwards *et al*. disclosed a solid-phase type approximation by the stepwise synthesis on a resin of the peptide D-Phe-Cys(Acm)-Phe-D-Trp(Boc)-Lys(Boc)-Thr(t-Bu)-Cys(Acm)-HMP-resin (SEQ. ID. NO. 1). Edwards et al., J. Med. Chem. 37, 3749-3757 (1994). Subsequently, the disulfide was prepared on the resin, and the resultant product released from the resin by means of aminolysis with threoninol. The total yield reported was only 14%.

Arano *et al*. carried out another solid phase method for DTPA-octreotide. Arano et al., Bioconjugate Chem., 8, 442-446 (1997). The iodine oxidation of the DTPA-peptide produced DTPA-D-Phe^{I}-octreotide in overall 31.8% yield based on the starting Fmoc-Thr(tBu)-ol-resin.

Wu *et al*. developed a synthetic method for octreotide, wherein the disulfide bond was formed by oxidation using a dilute solution of octreotide with air during 48 hours. Wu et al., Tetrahedron Letters, 39, 1783-1784 (1998). Lee *et al.* recently carried out a new method to anchor Thr(ol) (or Thr-ol) to a solid phase synthesis resin for preparation of octreotide. See, U.S. Patent No. 5,889,146. Fmoc-Thr(ol)-terephthal-acetal was loaded onto the resin and after construction of peptide chains using Fmoc chemistry, the cyclization of the peptide was obtained on resin by oxidation with iodine. The cleavage of peptide-resin with trifluoroacetic acid, produced octreotide with an overall yield of >70% from the starting Fmoc-Thr(ol)-terephthal-acetal-resin. All of these procedures completed the cyclization of the octreotide either on totally deprotected peptide or on the resin.

Further cyclic, bridge cyclic, and straight-chain somatostatin analogues and methods for their preparation are described in U.S. Patent Nos. 4,310,518 and 4,235,886; European Patent Specifications EP-A-1295; 70,021; 113,209; 215,171; 203,031; 214,872; and 143,307; and Belgian Patent Specification BE-A-900,089.

Peptide purification may remove impurities caused by side-chain modification, the deletion or addition sequences, or racemization products. Counterions introduced during peptide synthesis are also a source of impurities. Peptides that contain at least one basic function in their sequence (Lys or Arg side chains, or N-terminal amine group) appear as salts and not as a free base during synthesis and purification. The peptide counterions typically comprise trifluoroacetic acid (TFA) or phosphates, among others. Acetate is a pharmaceutically acceptable counterion and often the choice to replace the counterions used during synthesis or purification of active pharmaceutical ingredients (APIs). Thus, at some point during the peptide synthesis, the counterion must be replaced.

Commonly, ion exchange columns replace counterions in the polypeptide salts; however, the procedure often requires additional purification steps and an additional purification system. Reversed phase high-performance liquid chromatography (RP-HPLC) significantly improves the purification of synthetic peptides. However, solvent system selection remains difficult as peptides behave differently to the same solvents or solvent combinations. Although it is known that RP-HPLC can be used to perform ion exchange, one drawback of this method is the need for large solvent volumes. Another drawback is the difficulty to reduce the amount of unwanted residual counter ions to a low enough level.

Despite the improvements for peptide purification, some purified peptides still contain undesired amounts of unacceptable counterions. Residual counterions are often difficult to remove without additional purification steps. The invention provides an alternative method to reduce the counterion amount using ion exchange on RP-HPLC.

### Summary of the Invention

One embodiment of the invention encompasses a process for purifying a peptide comprising loading a peptide onto a RP-HPLC column; washing the column with an aqueous solution of a pharmaceutically acceptable counterion salt; and eluting the peptide from the column with a solvent mixture of a organic solvent and an acid of the pharmaceutically acceptable counterion, wherein the aqueous solution has a pH of at least about 6. The peptide may be cyclic or non-cyclic. The peptide may be vasopressin, atosiban, terlipressin, felypressin, ornipressin, pramlintide, AOD-9604, vapreotide, somatostatin, lanreotide, octreotide, eptifibatide, desmopressin, calcitonin salmon, oxytocin, or nesiritide. Preferably, the peptide is octreotide, desmopressin, calcitonin salmon, nesiritide, or eptifibatide.

In one embodiment, the pharmaceutically acceptable counterion salt is ammonium acetate, ammonium citrate, or ammonium pamoate. The pH of the aqueous solution may be about 8. The pH of the aqueous solution may be adjusted by at least one base, wherein the base is ammonia, ammonium hydroxide, methylamine, ethylamine, dimethylamine, diethylamine, methylethylamine, trimethylamine, or triethylamine. Preferably, the base is ammonium hydroxide.

In yet another embodiment, the solvent mixture has a pH of less than about 6. The organic solvent may be at least one of acetonitrile, methanol, ethanol, isopropanol, or THF. Preferably, the organic solvent is acetonitrile. The acid of the pharmaceutically acceptable counterion may be acetic acid, citric acid, or pamoitic acid.

One embodiment of the invention encompasses an eluted peptide having no more than about 0.25% by weight of residual counterion. Another embodiment encompasses an eluted peptide having no more than about 200 parts per million of residual counterion.

The invention encompasses nesiritide citrate having no more than about 0.25% by weight of residual counterion. The invention encompasses also eptifibatide acetate having no more than about 0.25% by weight of residual counterion.

### Detailed Description of the Invention

The invention encompasses processes for purifying peptides using a counterion exchange column and reverse phase HPLC (RP-HPLC). The process comprises purifying a peptide by ion-exchange, where the counterion of the peptide is exchanged with a pharmaceutically acceptable counterion. Thereafter, the peptide is collected by eluting the peptide from the column. In one embodiment, the process comprises loading a peptide onto a RP-HPLC column; washing the column with an aqueous solution of a pharmaceutically acceptable counterion; and eluting the peptide from the column with a solvent mixture of an organic solvent and an acid of the pharmaceutically acceptable counterion, wherein the aqueous solution has a pH of at least about 6.

The process is based in part on determining a suitable mobile phase for the HPLC system, because the elution of each peptide will depend upon the used solution. In particular, the mobile phase is a combination of aqueous and organic solvents in a various percentages. The elution may be under gradient or isocratic conditions.

Peptides suitable for the process include cyclic or non-cyclic peptides. The peptide may be in the form of a free acid or the salt of the corresponding acid. Suitable counterions used in the peptide salts include, but are not limited to, TFA, TEAP, hydrofluoric, hydrobromic, or suitable phosphate counterions. In particular, the peptides include, but are not limited to, vasopressin, atosiban, terlipressin, felypressin, omipressin, pramlintide, AOD-9604, vapreotide, somatostatin, lanreotide, octreotide, eptifibatide, desmopressin, calcitonin salmon, oxytocin, or nesiritide. Preferably, the peptides include octreotide, desmopressin, calcitonin salmon, nesiritide, or eptifibatide.

During the loading step, the peptide may be loaded as a solution comprising a peptide and a solvent. The peptide solution typically comprises the peptide diluted with water. However, the peptide may be loaded neat, i.e., without dilution.

Typically, the HPLC columns used in the process include standard HPLC columns such as silica base derivatized with oligomeric carbon chains. HPLC columns include, but are not limited to, C4 columns, octadecyl silica (C18), or octyl silica (C8) linked columns. Small to medium sized peptides, i.e., peptides having 5 to 50 residues are purified using octadecyl silica (C18), or octyl silica (C8) linked columns. Larger or more hydrophobic peptides are purified with C4 columns. Most preferably, the column used is C₁₈ RP-HPLC column.

The aqueous solution used in the washing step should be able to replace the peptide counterion with a pharmaceutically acceptable counterion. As used herein, the term "pharmaceutically acceptable counterion" refers to counterions that produce the pharmaceutically acceptable salt forms of the peptides. Examples of pharmaceutically acceptable counterions include, but are not limited to, acetate, ascorbate, benzenesulfonate, benzoate, bicarbonate, bisulfate, bitartrate, borate, camsylate, carbonate, citrate, dihydrochloride, methanesulfonate, ethanesulfonate, p-toluenesulfonate, cyclohexylsulfamate, quinate, edetate, edisylate, estolate, esylate, fumaxate, gluconate, glutamate, glycerophophates, hydrobromide, hydrochloride, hydroxynaphthoate, lactate, lactobionate, laurate, malate, maleate, mandelate, mesylate, mucate, napsylate, nitrate, n-methylglucamine, oleate, oxalate, palmoates, pamoate (embonate), palmitate, pantothenate, perchlorate, phosphate/diphosphate, polygalacturonate, salicylate, stearate, succinate, sulfate, sulfamate, subacetate, tannate, tartrate, tosylate, or valerate. Preferably, the pharmaceutically acceptable counterion is acetate, citrate, or pamoate.

Salt solutions of pharmaceutically acceptable counterions may be used in the washing step. Preferred pharmaceutically acceptable counterion salts include, but are not limited to, ammonium acetate, ammonium citrate, or ammonium pamoate. Preferably, a buffer gradient is used. In addition, any salt of an organic or inorganic acid can be used to replace the counterion. Similarly, any cation other than the ammonium cation may be used. Acids of pharmaceutically acceptable salts include, but are not limited to, acetic acid, citric acid, or pamoitic acid.

Typically, the aqueous solution of the washing step may have a concentration of from about 0.1 M to about 2 M. Preferably, the aqueous solution of the washing step has a concentration of about 0.2 M to about 1 M, and more preferably a concentration of about 0.5 M.

During the washing step, the pH of the aqueous solution is typically at least about 6. When a lower pH is used, the ion exchange is less effective and takes much more time and volume of the mobile phase required. Preferably, the pH of the aqueous solution during the washing of step is about 8. For columns that do not use silica gel or modified silica RP columns, a higher pH range is preferred rather than the pH stated for regular silica gel columns, such as a pH of 10. For example, the Luna sorbent column (Phenomenex) is stable at a pH of about 10. The pH for RP-polymer based columns, such as the PLRP-S column (by Polymer Laboratories) can be at about 10 or even higher, depending on stability of the specific peptide.

The pH of the aqueous solution in the washing step may be adjusted using a base. To avoid additional purification steps to remove non-volatile bases, it is preferred to adjust the pH using at least one volatile base. Examples of volatile bases include, but are not limited to, at least one of ammonia, ammonium hydroxide, methylamine, ethylamine, dimethylamine, diethylamine, methylethylamine, trimethylamine, or triethylamine. Preferably, the base is ammonium hydroxide. Salts of volatile bases may be used either alone or with the volatile base. For example, salts of volatile bases include, but are not limited to, ammonium acetate. Alternatively, the pH may be adjusted with inorganic bases or salts thereof, such as sodium, potassium, etc.

The eluting step is carried out using a solvent system capable of removing the peptide from the column. With little or no experimentation one of skill in the art will can easily determine the ratio of the solvents depending on the specific peptide and the purpose of the elution. The eluant used in the eluting step comprises at least one organic solvent and an acid of the pharmaceutically acceptable counterion. The eluant may be a mixture of the organic solvent and the acid in various proportions. For example, if the organic solvent is acetonitrile, then a concentration of about 50% may be used if the purpose is to remove the peptide from the column. Alternatively, a gradient of acetonitrile and aqueous acid may be used if the goal is to also further purify the peptide. A buffer gradient of the eluant is preferably used. Preferably, the pH of the eluant during the elution step is less than about 6.

The organic solvent includes, but is not limited to, at least one of acetonitrile, methanol, ethanol, isopropanol, or THF. The most preferred organic solvent is acetonitrile. The acid of the pharmaceutically acceptable ion is the acid of any of the pharmaceutically acceptable counterions described above. The selection of acid is dependent on the used pharmaceutically acceptable counterion.

Examples of systems used in HPLC include those involving a TFA system (acetonitrile/water 0.1% TFA) and a more ion-pairing system involving triethylammonium phosphate system (acetonitrile/TEAP buffered to a specific pH).

The process yields a peptide having residual counterion in no more than about 0.25% by weight. Alternatively, the purification process yields a peptide containing the residual counterion in no more than about 200 parts per million. Preferably, the counterion is TFA.

The residual counterion level may be determined by an Ion Chromatography (IC) method, such as DIONEX Application Note 115, or by other known methods such as HPLC or GC. As used herein, the term "residual counterion" is the counterion of the peptide used during the synthesis or purification of the peptide and present in the loading step, which is subsequently replaced by a pharmaceutically acceptable counterion.

Another embodiment of the invention encompasses nesiritide citrate having no more than about 0.25% by weight of residual counterion. The invention also encompasses eptifibatide acetate having no more than about 0.25% by weight of residual counterion.

While the present invention is described with respect to particular examples and preferred embodiments, it is understood that the present invention is not limited to these examples and embodiments. The present invention as claimed therefore includes variations from the particular examples and preferred embodiments described herein, as will be apparent to one of skill in the art.

### Examples

### Example 1: Preparation of H-Cys-Tyr-Phe-Gln-Asn-Cys(Acm)-Pro-Arg-Gly-NH₂ (SEQ. ID. NO.2) (Vasopressin precursor)

Synthesis of the peptide is carried out by a regular stepwise Fmoc SPPS (solid phase peptide synthesis) procedure starting from Rink amide resin (50 g). After removal of the Fmoc protecting group from the resin the first amino acid (Fmoc-Gly) is loaded on the resin in a regular coupling step to provide loading of about 0.7 mmol/g. After washing of the resin and removal of the Fmoc protecting group the second amino acid (Fmoc-Arg(Pbf)) is introduced to start the second coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by ninhydrine test. After washing of the resin, the Fmoc protecting group on the α-amine is removed with 20% piperidine in DMF for 20 min. These steps are repeated each time with another amino acid according to peptide sequence. All amino acids used are Fmoc-N^{α} protected. Trifunctional amino acids are side chain protected as follows: Tyr(tBu), Arg(Pbf), Cys(Acm) and Cys(Trt). Asn and Gln are used unprotected on the amide group. Three equivalents of the activated amino acids are employed in the coupling reactions. At the end of the synthesis the peptide-resin is washed with DMF, followed by DCM, and dried under vacuum to obtain 110 g dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether, filtered and dried in vacuum to obtain 36 g product. Residual TFA <0.25%.

### Example 2: Preparation of Vasopressin

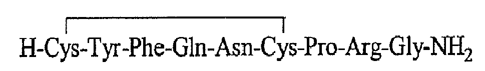

H-Cys-Tyr-Phe-Gln-Asn-Cys(Acm)-Pro-Arg-Gly-NH₂ (SEQ. ID. NO. 2) crude peptide (36 g, prepared as described in Example 1) is purified on preparative C₁₈ RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C₁₈ RP-HPLC column and purified to obtain fractions containing vasopressin trifluoroacetate at a purity of >98.5%. The fractions are treated to replace TFA ion by acetate, collected and lyophilized to obtain final dry peptide 11 g (>99.0% pure).

### Example 3: Preparation of H-Lys-Cys-Asn-Thr-Ala-Thr-Cys(Acm)-Ala-Thr-Gln-Arg-Leu-Ala-Asn-Phe-Leu-Val-His-Ser-Ser-Asn-Asn-Phe-Gly-Pro-Ile-Leu-Pro-Pro-Thr-Asn-Val-Gly-Ser-Asn-Thr-Tyr-NH₂ (SEO. ID. NO. 7) (Pramlintide precursor)

Synthesis of the peptide is carried out by a regular stepwise Fmoc SPPS (solid phase peptide synthesis) procedure starting from Rink amide resin as described in Example 1. All amino acids used are Fmoc-N^{α} protected. Trifunctional amino acids are side chain protected as follows: Lys(Boc), Thr(tBu), His(Trt), Ser(tBu), Tyr(tBu), Arg(Pbf), Cys(Acm) and Cys(Trt). Asn and Gln are used unprotected or as Trt protected on the amide group. Three equivalents of the activated amino acids are employed in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether (MTBE), filtered and dried in vacuum.

### Example 4: Preparation of H-Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Pro-Ile-Leu-Pro-Pro-Thr-Asn-Val-Gly-Ser-Asn-Thr-Tyr-NH₂ cyclic (2-7)-disulfide, acetate (SEQ. ID. NO. 7).

The crude semi-protected peptide, prepared as described in Example 3, is purified on preparative C₁₈ RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C₁₈ RP-HPLC column and purified to obtain fractions containing Pramlintide trifluoroacetate at a purity of >97.5%. The fractions are treated to replace TFA ion by acetate, collected and lyophilized to obtain final Pramlintide acetate. Residual TFA <0.25%.

### Example 5: Preparation of H-Tyr-Leu-Arg-Ile-Val-Gln-Cys-Arg-Ser-Val-Glu-Gly-Ser-Cys(Acm)-Gly-Phe-OH (SEQ. ID. NO. 8) (AOD-9604 precursor)

Synthesis of the peptide is carried out by a regular stepwise Fmoc SPPS (solid phase peptide synthesis) procedure starting from 2-Cl-Trt resin. The first amino acid (Fmoc-Phe) is loaded on the resin in a preliminary step to provide loading of about 0.7 mmol/g. After washing of the resin the Fmoc group is removed by treatment with piperidine/DMF solution and second amino acid (Fmoc-Gly) is introduced to start the first coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by ninhydrine test. After washing of the resin, the Fmoc protecting group on the α-amine is removed with 20% piperidine in DMF for 20 min. These steps were repeated each time with another amino acid according to peptide sequence. All amino acids used are Fmoc-N^{α} protected. Trifunctional amino acids are side chain protected as follows: Tyr(tBu), Arg(Pbf), Ser(tBu), Cys(Trt) and Cys(Acm). Three equivalents of the activated amino acids are employed in the coupling reactions. At the end of the synthesis the peptide-resin is washed with DMF, followed by DCM, and dried under vacuum to obtain dry peptide-resin.

The peptide, prepared as described above, is cleaved from the resin accompanied with simultaneous deprotection of acid-labile protecting groups using a 95% TFA, 2.5% TIS, 2.5% EDT solution for 2 hours at room temperature. The product is precipitated by the addition of 10 volumes of ether, filtered and dried in vacuum.

### Example 6: Preparation of AOD-9604

H-Tyr-Leu-Arg-Ile-Val-Gln-Cys-Arg-Ser-Val-Glu-Gly-Ser-Cys-Gly-Phe-OH

H-Tyr-Leu-Arg-Ile-Val-Gln-Cys-Arg-Ser-Val-Glu-Gly-Ser-Cys(Acm)-Gly-Phe-OH (SEQ. ID. NO. 8) crude peptide, prepared as described in Example 5, is purified on preparative C₁₈ RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C₁₈ RP-HPLC column and purified to obtain fractions containing peptide at a purity of >98.5%. The fractions are collected and lyophilized to obtain final dry peptide. Residual TFA<0.25%.

### Example 7: Preparation of H-Ala-Gly-Cys-Lys-Asn-Phe-Phe-Trp-Lys-Thr-Ser-Cys(Acm)-OH (SEQ. ID. NO. 10) (Somatostatin precursor)

Synthesis of the peptide is carried out by a regular stepwise Fmoc SPPS (solid phase peptide synthesis) procedure starting from 2-Cl-Trt resin. The first amino acid (Fmoc-Cys(Acm)) is loaded on the resin in a preliminary step to provide loading of about 0.7 mmol/g. After washing of the resin the Fmoc group is removed by treatment with piperidine/DMF solution and second amino acid (Fmoc-Ser(tBu)) is introduced to start the first coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by ninhydrine test. After washing of the resin, the Fmoc protecting group on the α-amine is removed with 20% piperidine in DMF for 20 min. These steps were repeated each time with another amino acid according to peptide sequence. All amino acids used are Fmoc-N^{α} protected. Trifunctional amino acids are side chain protected as follows: Lys(Boc), Thr(tBu), Ser(tBu), Cys(Trt) and Cys(Acm). Three equivalents of the activated amino acids are employed in the coupling reactions. At the end of the synthesis the peptide-resin is washed with DMF, followed by DCM, and dried under vacuum to obtain dry peptide-resin.

The peptide, prepared as described above, is cleaved from the resin accompanied with simultaneous deprotection of acid-labile protecting groups using a 95% TFA, 2.5% TIS, 2.5% EDT solution for 2 hours at room temperature. The product is precipitated by the addition of 10 volumes of ether, filtered and dried in vacuum.

### Example 8: Preparation of Somatostatin

H-Ala-Gly-Cys-Lys-Asn-Phe-Phe-Trp-Lys-Thr-Phe-Thr-Ser-Cys-OH

H-Ala-Gly-Cys-Lys-Asn-Phe-Phe-Trp-Lys-Thr-Ser-Cys(Acm)-OH (SEQ. ID. NO. 10) crude peptide, prepared as described in Example 7, is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C18 RP-HPLC column and purified to obtain fractions containing peptide at a purity of >98.5%. After treatment to replace counterion to acetate the fractions are collected and lyophilized to obtain final dry peptide. Residual TFA <0.25%.

### Example 9: Preparation of H-D-Naph-Cys-Tyr-D-Try-Lys-Val-Cys(Acm)-Thr-NH₂ (SEQ. ID. NO. 11) (Lanreotide precursor)

Synthesis of the peptide is carried out by a regular stepwise Fmoc SPPS (solid phase peptide synthesis) procedure starting from Rink amide resin. After removal of the Fmoc protecting group from the resin the first amino acid (Fmoc-Thr(tBu)) is loaded on the resin in a regular coupling step to provide loading of about 0.7 mmol/g. After washing of the resin and removal of the Fmoc protecting group the second amino acid (Fmoc-Gys(Acm)) is introduced to start the second coupling step; Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by ninhydrine test. After washing of the resin, the Fmoc protecting group on the α-amine is removed with 20% piperidine in DMF for 20 min. These steps are repeated each time with another amino acid according to peptide sequence. All amino acids used are Fmoc-N^{α} protected. Trifunctional amino acids are side chain protected as follows: Tyr(tBu), Lys(Boc), Thr(tBu), Cys(Acm), and Cys(Trt). Three equivalents of the activated amino acids are used in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether, filtered and dried in vacuum to obtain crude product.

### Example 10: Preparation of Lanreotide

H-D-Naph-Cys-Tyr-D-Trp-Lys-Val-Cys-Thr-NH2

H-D-Naph-Cys-Tyr-D-Trp-Lys-Val-Cys(Acm)-Thr-NH₂ (SEQ. ID. NO. 11) crude peptide prepared as described in Example 9 is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C18 RP-HPLC column and purified to obtain fractions containing Lanreotide at a purity of >98.5%. The fractions are treated to replace counterion by acetate, collected and lyophilized to obtain final dry peptide. Residual TFA <0.25%.

### Example 11: Preparation of Mpa-D-Tyr(Et)-Ile-Thr-Asn-Cys(Acm)-Pro-Orn-Gly-NH₂ (SEQ. ID. NO. 3) (Atosiban precursor)

Synthesis of the peptide is carried out by a regular stepwise Fmoc SPPS (solid phase peptide synthesis) procedure starting from Rink amide resin. After removal of the Fmoc protecting group from the resin the first amino acid (Fmoc-Gly) is loaded on the resin in a regular coupling step to provide loading of about 0.7 mmol/g. After washing of the resin and removal of the Fmoc protecting group the second amino acid (Fmoc-Om(Boc)) is introduced to start the second coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by Ninhydrine test. After washing of the resin, the Fmoc protecting group on the α-amine is removed with 20% piperidine in DMF for 20 min. These steps are repeated each time with another amino acid according to peptide sequence. All amino acids used are Fmoc-N" protected. Trifunctional amino acids are side chain protected as follows: Orn(Boc), Thr(tBu), Cys(Acm), and Mpa(Trt). Three equivalents of the activated amino acids are used in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether, filtered and dried in vacuum to obtain crude product.

### Example 12: Preparation of Atosiban

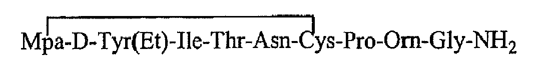
Mpa-D-Tyr(Et)-Ile-Thr-Asn-Cys-Pro-Orn-Gly-NH2

Mpa-D-Tyr(Et)-Ile-Thr-Asn-Cys(Acm)-Pro-Orn-Gly-NH₂ (SEQ. ID. NO. 3) crude peptide prepared as described in Example 11 is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C18 RP-HPLC column and purified to obtain fractions containing Atosiban at a purity of >98.5%. The fractions are treated to replace counterion, collected and lyophilized to obtain final dry peptide. Residual TFA <0.25%.

### Example 13: Preparation of H-Gly-Gly-Gly-Cys-Tyr-Phe-Gln-Asn-Cys(Acm)-Pro-Lys-Gly-NH₂ (SEQ. ID. NO. 4) (Terlipressin precursor)

Synthesis of the peptide is carried out by a regular stepwise Fmoc SPPS (solid phase peptide synthesis) procedure starting from Rink amide resin. After removal of the Fmoc protecting group from the resin the first amino acid (Fmoc-Gly) is loaded on the resin in a regular coupling step to provide loading of about 0.7 mmol/g. After washing of the resin and removal of the Fmoc protecting group the second amino acid (Fmoc-Lys(Boc)) is introduced to start the second coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by Ninhydrine test. After washing of the resin, the Fmoc protecting group on the α-amine is removed with 20% piperidine in DMF for 20 min. These steps are repeated each time with another amino acid according to peptide sequence. All amino acids used are Fmoc-N^{α}-protected. Trifunctional amino acids are side chain protected as follows: Lys(Boc), Tyr(tBu), Cys(Acm), and Cys(Trt). Three equivalents of the activated amino acids are used in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether, filtered and dried in vacuum to obtain crude product.

### Example 14: Preparation of Terlipressin

H-Gly-Gly-Gly-Cys-Tyr-Phe-Gln-Asn-Cys-Pro-Lys-Gly-NH2

H-Gly-Gly-Gly-Cys-Tyr-Phe-Gln-Asn-Cys(Acm)-Pro-Lys-Gly-NH₂ (SEQ. ID. NO. 4) crude peptide prepared as described in Example 13 is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C 18 RP-HPLC column and purified to obtain fractions containing Terlipressin at a purity of >98.5%. The fractions are treated to replace counterion, collected and lyophilized to obtain final dry peptide. Residual TFA <0.25%.

### Example 15: Preparation of H-Cys-Phe-Phe-Gln-Asn-Cys(Acm)-Pro-Lys-Gly-NH₂ (SEQ. ID NO. 5) (Felypressin precursor)

Synthesis of the peptide is carried out by a regular stepwise Fmoc SPPS (solid phase peptide synthesis) procedure starting from Rink amide resin. After removal of the Fmoc protecting group from the resin the first amino acid (Fmoc-Gly) is loaded on the resin in a regular coupling step to provide loading of about 0.7 mmol/g. After washing of the resin and removal of the Fmoc protecting group the second amino acid (Fmoc-Lys(Boc)) is introduced to start the second coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by Ninhydrine tesL After washing of the resin, the Fmoc protecting group on the α-amine is removed with 20% piperidine in DMF for 20 min. These steps are repeated each time with another amino acid according to peptide sequence. All amino acids used are Fmoc-N^{α} protected. Trifunctional amino acids are side chain protected as follows: Lys(Boc), Cys(Acm) and Cys(Trt). Three equivalents of the activated amino acids are used in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether (MTBE), filtered and dried in vacuum to obtain crude product.

### Example 16: Preparation of Felypressin

H-Cys-Phe-Phe-Gln-Asn-Cys-Pro-Lys-Gly-NH2

H-Cys-Phe-Phe-Gln-Asn-Cys(Acm)-Pro-Lys-Gly-NH₂ (SEQ. ID. NO. 5) crude peptide prepared as described in Example 15 is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C18 RP-HPLC column and purified to obtain fractions containing Felypressin at a purity of >98.5%. The fractions are treated to replace counterion, collected and lyophilized to obtain final dry peptide. Residual TFA <0.25%.

### Example 17: Preparation of H-Cys-Tyr-Phe-Gln-Asn-Cys(Acm)-Pro-Orn-Gly-NH₂ (SEQ. ID. NO. 6) (Ornipressin precursor)

Synthesis of the peptide is carried out by a regular stepwise Fmoc SPPS (solid phase peptide synthesis) procedure starting from Rink amide resin. After removal of the Fmoc protecting group from the resin the first amino acid (Fmoc-Gly) is loaded on the resin in a regular coupling step to provide loading of about 0.7 mmol/g. After washing of the resin and removal of the Fmoc protecting group the second amino acid (Fmoc-Orn(Boc)) is introduced to start the second coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by ninhydrine test. After washing of the resin, the Fmoc protecting group on the α-amine is removed with 20% piperidine in DMF for 20 min. These steps are repeated each time with another amino acid according to peptide sequence. All amino acids used are Fmoc-N^{α} protected. Trifunctional amino acids are side chain protected as follows: Om(Boc), Tyr(tBu), Cys(Acm) and Cys(Trt). Three equivalents of the activated amino acids are used in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether, filtered and dried in vacuum to obtain crude product.

### Example 18: Preparation of Ornipressin

H-Cys-Tyr-Phe-Gln-Asn-Cys-Pro-Orn-Gly-NH2

H-Cys-Tyr-Phe-Gln-Asn-Cys(Acm)-Pro-Orn-Gly-NH₂ (SEQ. ID. NO. 6) crude peptide prepared as described in Example 17 is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C18 RP-HPLC column and purified to obtain fractions containing Ornipressin at a purity of >98.5%. The fractions are treated to replace counterion, collected and lyophilized to obtain final dry peptide. Residual TFA <0.25%.

### Example 19: Preparation of H-D-Phe-Cys-Tyr-D-Trp-Lys-Cys(Acm)-Trp-NH₂ (SEQ. ID. NO. 9) (Vapreotide precursor)

Synthesis of the peptide is carried out by a regular stepwise Fmoc SPPS (solid phase peptide synthesis) procedure starting from Rink amide resin. After removal of the Fmoc protecting group from the resin the first amino acid (Fmoc-Trp) is loaded on the resin in a regular coupling step to provide loading of about 0.7 mmol/g. After washing of the resin and removal of the Fmoc protecting group the second amino acid (Fmoc-Cys(Acm)) is introduced to start the second coupling step. Fmoc protected amino acids are activated in situ using TBTU/HOBt (N-hydroxybenzotriazole) and subsequently coupled to the resin for 50 minutes. Diisopropylethylamine or collidine are used during coupling as an organic base. Completion of the coupling is indicated by Ninhydrine test. After washing of the resin, the Fmoc protecting group on the α-amine is removed with 20% piperidine in DMF for 20 min. These steps are repeated each time with another amino acid according to peptide sequence. All amino acids used are Fmoc-N^{α} protected. Trifunctional amino acids are side chain protected as follows: Lys(Boc), Tyr(tBu), Cys(Acm) and Cys(Trt). Three equivalents of the activated amino acids are used in the coupling reactions. At the end of the synthesis the peptide-resin is washed with DMF, followed by DCM, and dried under vacuum to obtain dry peptide-resin.

The peptide, prepared as described above, is cleaved from the resin together with removal of acid-labile protecting groups using a 95% TFA, 2.5% TIS, 2.5% EDT solution for 2 hours at room temperature. The product is precipitated by the addition of 10 volumes of ether, filtered and dried in vacuum to obtain crude product.

### Example 20: Preparation of Vapreotide

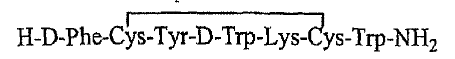
H-D-Phe-Cys-Tyr-D-Trp-Lys-Cys-Trp-NH2

H-D-Phe-Cys-Tyr-D-Trp-Lys-Cys(Acm)-Trp-NH₂ (SEQ. ID. NO. 9) crude peptide prepared as described in Example 19 is purified on preparative C18 RP-HPLC column. Fractions containing >95% pure product are combined and diluted to concentrations of about 1 g/L. An equimolar amount of iodine in acetic acid is added under vigorous mixing at room temperature and subsequently excess iodine is neutralized by small amount of ascorbic acid. The resulting solution is loaded on a C18 RP-HPLC column and purified to obtain fractions containing Vapreotide at a purity of >98.5%. The fractions are treated to replace counterion, collected and lyophilized to obtain final dry peptide. Residual TFA <0.25%.

### Example 21: Preparation of H-D-Phe-Cys-Phe-D-Trp-Lys-Thr-Cys(Acm)-Thr-ol (SEQ. ID. NO. 1) (Octreotide precursor)

Synthesis of the peptide was carried out by a stepwise Fmoc SPPS (solid phase peptide synthesis) procedure starting from Thr(t-Bu)-ol-2-Cl-Trt resin (250 g, loading of 0.7 mmol on 1 g of preloaded resin). After washing of the resin the second amino acid (Fmoc-Cys(Acm)) was introduced to start the first coupling step. Fmoc protected amino acid was activated in situ using TBTU/HOBt (N-hydroxybenzotriazole) and subsequently coupled to the resin for 50 minutes. Diisopropylethylamine or collidine was used during coupling as an organic base. Completion of the coupling was indicated by Ninhydrine test. After washing of the resin, the Fmoc protecting group on the ∀-amine was removed with 20% piperidine in DMF for 20 min. These steps were repeated each time with another amino acid according to peptide sequence. All amino acids used were Fmoc-N^{∀} protected except the last amino acid in the sequence, Boc-D-Phe. Trifunctional amino acids were side chain protected as follows: Thr(t-Bu), Cys(Trt), Cys(Acm), and Lys(Boc). Three equivalents of the activated amino acids were employed in the coupling reactions. At the end of the synthesis the peptide-resin was washed with DMF, followed by DCM, and dried under vacuum to obtain 510 g dry peptide-resin.

The peptide, prepared as described above, was cleaved from the resin using a 95% TFA, 2.5% TIS, 2.5% EDT solution for 2 hours at room temperature. The product was precipitated by the addition of 10 volumes of ether (MTBE), filtered and dried in vacuum to obtain 201.7 g powder. It was identified by LC/MS as H-D-Phe-Cys-Phe-D-Trp-Lys-Thr-Cys(Acm)-Thr-ol.

### Example 22: Preparation of Octreotide

D-Phe—Cys—Phe—D-Trp—Lys—Thr—Cys—Thr-ol

H-D-Phe-Cys-Phe-D-Trp-Lys-Thr-Cys(Acm)-Thr-ol (SEQ. ID. NO. 1) crude peptide (200 g, prepared as described in example (21) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing octreotide trifluoroacetate at a purity of >98.5%. After treatment to replace trifluoroacetate, the fractions were collected and lyophilized to obtain final dry peptide (130 g). Residual TFA <0.25%.

### Example 23: Preparation of Mpa-Har-Gly-Asp-Trp-Pro-Cys(Acm)-NH₂ (SEQ. ID. NO. 12) (Eptifibatide precursor)

Synthesis of the peptide was carried out by a regular stepwise Fmoc SPPS (solid phase peptide synthesis) procedure starting from 2-Cl-Trt resin (50 g). The first amino acid (Fmoc-Cys(Acm)) was loaded onto the resin in a preliminary step to provide loading of about 0.7 mmol/g. After resin washing, a second amino acid (Fmoc-Pro) was introduced to start the first coupling step. Fmoc protected amino acid was activated in situ using TBTU/HOBt and subsequently coupled to the resin for 50 minutes. Diisopropylethylamine or Collidine were used during coupling as an organic base. Completion of the coupling was indicated by ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected except the last building block in the sequence, Trt-Mpa. Trifunctional amino acids were side chain protected as follows: Asp(tBu), Har(Pbf), and Cys(Acm). Three equivalents of the activated amino acids were employed in the coupling reactions. At the end of the synthesis the peptide-resin was washed with DMF, followed by DCM, and dried under vacuum to obtain 80 g dry peptide-resin.

The peptide, prepared as described above, was cleaved from the resin by washing with a solution of 1% TFA in DCM. The resulted solution was neutralized by addition of DIPEA and concentrated to about 10% peptide content. Amidation of the C-terminus was achieved by activation of the carboxy terminus with DCC/HOBt and coupling with ammonia solution in IPA. After removal of the solvent the protected peptide was precipitated in ether and dried. The protecting groups were removed using a 95% TFA, 2.5% TIS, 2.5% EDT solution for 2 hours at room temperature. The product was precipitated by the addition of 10 volumes of ether, filtered and dried in vacuum to obtain 30 g product.

### Example 24: Preparation of Eptifibatide

Mpa—Har—Gly—Asp—Trp—Pro—Cys—NH2

Mpa-Har-Gly-Asp-Trp-Pro-Cys(Acm)-NH₂ crude peptide (SEQ. ID. NO. 12) (30 g, prepared as described in example 23) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing eptifibatide trifluoroacetate at a purity of >98.5%. The fractions were collected and lyophilized to obtain final dry peptide 6.9 g (>98.5% pure). Residual TFA <0.25%.

### Example 25: Preparation of Mpa-Tyr-Phe-Gln-Asn-Cys(Acm)-Pro-D-Arg-Gly-NH₂ (Desmopressin precursor) by SPPS method

Synthesis of the peptide was carried out by a regular stepwise Fmoc SPPS procedure starting from Rink amide resin (200 g). The first amino acid (Fmoc-Gly) was loaded on the resin by a regular coupling procedure after removal of the Fmoc group from the resin. After washing of the resin the second amino acid (Fmoc-D-Arg(Pbf)) was introduced to continue sequence elongation. Fmoc protected amino acids were activated in situ using TBTU/HOBt and subsequently coupled to the resin over about 50 minutes. Diisopropylethylamine or collidine were used during coupling as an organic base. Completion of the coupling was indicated by ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected except the last building block in the sequence, Trt-Mpa. Trifunctional amino acids were side chain protected as follows: Gln(Trt), D-Arg(Pbf), Tyr(tBu) and Cys(Acm). Three equivalents of the activated amino acids were employed in the coupling reactions. At the end of the synthesis the peptide-resin was washed with DMF, followed by DCM, and dried under vacuum to obtain 460g dry peptide-resin.

The peptide, prepared as described above, was cleaved from the resin using a 89% TFA, 5.0% Phenol, 1.0% TIS, 2.5% EDT, 2.5% water solution for 1.5 hours at room temperature. The product was precipitated by the addition of 10 volumes of ether, filtered and dried in vacuum to obtain 115.0 g powder. It was identified by LC/MS as Mpa-Tyr-Phe-Gln-Asn-Cys(Acm)-Pro-D-Arg-Gly-NH₂.

### Example 26: Preparation of Mpa-Tyr-Php-Gln-Asn-Cys(Acm)-Pro-D-Arg-Gly-NH₂ (SEQ. ID. NO. 13) (Desmopressin precursor) in solution method

Synthesis of the peptide is carried out by a regular stepwise "solution synthesis" method. The second amino acid (Fmoc-D-Arg(Pbf)-OH) is dissolved in DMF and preactivated by addition of TBTU/HOBt in the presence of DIPEA. The first amino acid (Gly-NH₂) is dissolved in DMF, is added, and the reaction continues for about 1 h at room temperature. DMF is removed under low pressure and the residue is dissolved in ethylacetate. The organic solution is washed several times with aqueous HCl (1N), water and, NaHCO₃ (5%). After the solution is dried over Na₂SO₄, the solvent is evaporated to obtain Fmoc-D-Arg(Pbf)-Gly-NH₂. Fmoc group is removed by dissolution in piperidine/DMF (20%). The solution is concentrated and the crude di-peptide is precipitated in cold ether. By a similar procedure the rest of amino acids are added sequentially to obtain final protected linear peptide. Fmoc protected amino acids are activated in situ using TBTU/HOBt and subsequently coupled to the growing peptide chain. Diisopropylethylamine or collidine are used during coupling as an organic base. Completion of the coupling is determined by HPLC or TLC test. These steps are repeated each time with another amino acid according to the peptide sequence. All amino acids used are Fmoc-N^{α} protected except the last building block in the sequence, Trt-Mpa. Trifunctional amino acids are side chain protected as follows: Gln(Trt), D-Arg(Pbf), Tyr(tBu) and Cys(Acm).

The peptide, prepared as described above, is deprotected from its acid-labile protecting groups using a 91.5% TFA, 1.0% TIS, 2.5% EDT, 5.0% water solution for 1.5 hours at room temperature. The crude product, Mpa-Tyr-Phe-Gln-Asn-Cys(Acm)-Pro-D-Arg-Gly-NH₂, is precipitated by the addition of 10 volumes of ether, filtered, and dried in a vacuum to obtain fine powder. The product is identified by LC/MS.

### Example 27: Preparation of Desmopressin

Mpa—Tyr—Phe—Gln—Asn—Cys—Pro—D-Arg—Gly—NH2

Mpa-Tyr-Phe-Gln-Asn-Cys(Acm)-Pro-D-Arg-Gly-NH₂ (SEQ. ID. NO. 13) crude peptide (115 g, prepared as described in Example 25) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing desmopressin trifluoroacetate at a purity of >98.5%. After exchange of the counterion to acetate the fractions were collected and lyophilized to obtain final dry peptide 37.2 g. The peptide was >99.5% pure (by HPLC). Residual TFA <0.25%.

### Example 28: Preparation of Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂ (SEQ. ID. NO. 14) (Calcitonin salmon precursor)

Synthesis of the peptide was carried out by a regular stepwise Fmoc SPPS procedure starting from Rink amide resin (200 g). The first amino acid (Fmoc-Pro) was loaded on the resin by a regular coupling procedure after removal of the Fmoc group from the resin. After washing of the resin the second amino acid (Fmoc-Thr(tBu) was introduced to continue sequence elongation. Fmoc protected amino acids were activated in situ using TBTU/HOBt and subsequently coupled to the resin during about 60 minutes. Diisopropylethylamine or collidine were used during coupling as an organic base. Completion of the coupling was indicated by ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected. Trifunctional amino acids were side chain protected as follows: Cys(Trt), Ser(tBu), Asn(Trt), Gln(Trt), Thr(tBu), Glu(tBu), His(Trt), Lys(Boc), Arg(Pbf), Tyr(tBu) and Cys(Acm). Three equivalents of the activated amino acids were employed in the coupling reactions. At the end of the synthesis the peptide-resin was washed with DMF, followed by DCM, and dried under vacuum to obtain 670 g dry peptide-resin.

The peptide, prepared as described above, was cleaved from the resin using a 94% TFA, 1.0% TIS, 2.5% EDT, 2.5% water solution for 1.5 hours at room temperature. The product was precipitated by the addition of 10 volumes of ether, filtered and dried in vacuum to obtain 345.0 g powder. It was identified by LC/MS as Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂.

### Example 29: Preparation of Calcitonin (salmon)

Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂ (SEQ. ID. NO. 14) crude peptide (345 g, prepared as described in Example 28) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing calcitonin trifluoroacetate at a purity of >98.5%. After exchange of the counterion to acetate the fractions were collected and lyophilized to obtain final dry peptide 64 g. The peptide was >99.5% pure (by HPLC). Residual TFA <0.25%.

### Example 30: Preparation of H-Cys(Acm)-Tyr-Ile-Gln-Asn-Cys-Pro-Leu-Gly-NH₂ (SEQ. ID. NO. 15) (Oxytocin precursor)

Synthesis of the peptide was carried out by a regular stepwise Fmoc SPPS (solid phase peptide synthesis) procedure starting from Rink amide resin. After removal of the Fmoc protecting group from the resin, the first amino acid (Fmoc-Gly) was loaded on the resin in a regular coupling step to provide loading of about 0.7 mmol/g. After washing the resin and removing the Fmoc protecting group, the second amino acid (Fmoc-Leu) was introduced to start the second coupling step. Fmoc protected amino acids were activated in situ using TBTU/HOBt (N-hydroxybenzotriazole) and subsequently coupled to the resin for 50 minutes. Diisopropylethylamine or collidine were used during coupling as an organic base. Completion of the coupling was indicated by Ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected. Trifunctional amino acids were side chain protected as follows: Tyr(tBu), Cys(Acm) and Cys(Trt). Three equivalents of the activated amino acids were used in the coupling reactions. At the end of the synthesis the peptide-resin was washed with DMF, followed by DCM, and dried under vacuum to obtain dry peptide-resin.

The peptide, prepared as described above, was cleaved from the resin together with removal of acid-labile protecting groups using a 95% TFA, 2.5% TIS, 2.5% EDT solution for 2 hours at room temperature. The product was precipitated by the addition of 10 volumes of ether, filtered and dried in vacuum to obtain crude product.

### Example 31: Preparation of Oxytocin

H-Cys-Tyr-Ile-Gln-Asn-Cys-Pro-Leu-Gly-NH2

H-Cys(Acm)-Tyr-Ile-Gln-Asn-Cys-Pro-Leu-Gly-NH₂ (SEQ. ID. NO. 15) crude peptide prepared as described in Example 30 was purified on preparative C18 RP-HPLC column. Fractions containing >95% pure product were combined and diluted to concentrations of about 1 g/L. An equimolar amount of iodine in acetic acid was added under vigorous mixing at room temperature, and the excess iodine was neutralized by a small amount of ascorbic acid. The resulting solution was loaded on a C18 RP-HPLC column and purified to obtain fractions containing oxytocin at a purity of >98.5%. The fractions were treated to replace the counterion, collected and lyophilized to obtain final dry peptide. Residual TFA <0.25%.

### Example 32: Preparation of H-Ser-Pro-Lys-Met-Val-Gln-Gly-Ser-Gly-Cys-Phe-Gly-Arg-Lys-Met-Asp-Arg-Ile-Ser-Ser-Ser-Ser-Gly-Leu-Gly-Cys(Acm)-Lys-Val-Leu-Arg-Arg-His-OH (SEQ. ID. NO. 16) (Nesiritide precursor)

Synthesis of the peptide was carried out by a regular stepwise Fmoc SPPS (solid phase peptide synthesis) procedure starting from 2-Cl-Trt resin. The first amino acid (Fmoc-His(Trt)) was loaded on the resin in a preliminary step to provide loading of about 0.3 mmol/g. After washing the resin the Fmoc group was removed by treatment with piperidine/DMF solution, and a second amino acid (Fmoc-Arg(Pbf) was introduced to start the first coupling step. Fmoc protected amino acids were activated in situ using TBTU and HOBt (N-hydroxybenzotriazole) and subsequently coupled to the resin for 50 minutes. Diisopropylethylamine or collidine were used during coupling as an organic base. Completion of the coupling was indicated by ninhydrine test. After washing the resin, the Fmoc protecting group on the α-amine was removed with 20% piperidine in DMF for 20 min. These steps were repeated each time with another amino acid according to peptide sequence. All amino acids used are Fmoc-N^{α} protected. Trifunctional amino acids were side chain protected as follows: Lys(Boc), Arg(Pbf), Ser(tBu), Asp(tBu), His(Trt), Cys(Trt) and Cys(Acm). Three equivalents of the activated amino acids were employed in the coupling reactions. At the end of the synthesis the peptide-resin was washed with DMF, followed by DCM, and dried under vacuum to obtain dry peptide-resin.

The peptide, prepared as described above, was cleaved from the resin accompanied with simultaneous deprotection of acid-labile protecting groups using a 95% TFA, 2.5% TIS, 2.5% EDT solution for 2 hours at room temperature. The product was precipitated by the addition of 10 volumes of ether, filtered and dried in vacuum.

### Example 33: Preparation of Nesiritide H-Ser-Pro-Lys-Met-Val-Gln-Gly-Ser-Gly-Cys-Phe-Gly-Arg-Lys-Met- Asp-Arg-Ile-Ser-Ser-Ser-Ser-Gly-Leu-Gly-Cys-Lys-Val-Leu-Arg-Arg-His-OH (10,26 cyclic) citrate salt

H-Ser-Pro-Lys-Met-Val-Gln-Gly-Ser-Gly-Cys-Phe-Gly-Arg-Lys-Met-Asp-Arg-Ile-Ser-Ser-Ser-Ser-Giy-Leu-Gly-Cys(Acm)-Lys-Val-Leu-Arg-Arg-His-OH (SEQ. ID. NO. 16) crude peptide, prepared as described in Example 32, was purified on preparative C18 RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C18 RP-HPLC column and purified to obtain fractions containing peptide at a purity of >98.5%. After treatment to replace counterion to citrate the fractions were collected and lyophilized to obtain final dry peptide. Residual TFA <0.25%.

Other embodiments of the present invention are encompassed by the following number clauses:
1. A process for purifying a peptide comprising:
   a) loading a peptide onto a RP-HPLC column;
   b) washing the column with an aqueous solution of a pharmaceutically acceptable counterion salt; and
   c) eluting the peptide from the column with a solvent mixture of an organic solvent and an acid of the pharmaceutically acceptable counterion, wherein the aqueous solution has a pH of at least about 6.
2. The process according to clause 1, wherein the peptide is cyclic or non-cyclic.
3. The process according to clause 1, wherein the peptide is vasopressin, atosiban, terlipressin, felypressin, omipressin, pramlintide, AOD-9604, vapreotide, somatostatin, lanreotide, octreotide, eptifibatide, desmopressin, calcitonin salmon, oxytocin, or nesiritide.
4. The process according to clause 3, wherein the peptide is octreotide, desmopressin, calcitonin salmon, nesiritide, or eptifibatide.
5. The process according to clause 1, wherein the pharmaceutically acceptable counterion salt is ammonium acetate, ammonium citrate, or ammonium pamoate.
6. The process according to clause 1, wherein the pH of the aqueous solution is about 8.
7. The process according to clause 6, wherein the pH of the aqueous solution is adjusted by at least one base.
8. The process according to clause 7, wherein the base is ammonia, ammonium hydroxide, methylamine, ethylamine, dimethylamine, diethylamine, methylethylamine, trimethylamine, or triethylamine.
9. The process according to clause 7, wherein the base is ammonium hydroxide.
10. The process according to clause 1, wherein the solvent mixture has a pH of less than about 6.
11. The process according to clause 1, wherein the organic solvent is at least one of acetonitrile, methanol, ethanol, isopropanol, or THF.
12. The process according to clause 11, wherein the organic solvent is acetonitrile.
13. The process according to clause 1, wherein the acid of the pharmaceutically acceptable counterion is acetic acid, citric acid, or pamoitic acid.
14. The process according to clause 1, wherein the eluted peptide has no more than about 0.25% by weight of residual counterion.
15. The process according to clause 1, wherein the eluted peptide has no more than about 200 parts per million of residual counterion.
16. Nesiritide citrate having no more than about 0.25% by weight of residual counterion.
17. Eptifibatide acetate having no more than about 0.25% by weight of residual counterion.

## Claims

1. Atosiban or lanreotide having residual counterion in no more than about 0.25% by weight.

2. Atosiban or lanreotide having residual counterion in no more than about 200 parts per million.

3. The atosiban or lanreotide of claims 1 or 2 wherein the residual counterion is triflouoroacetic acid.

4. A peptide produced by a process for purifying a peptide by counterion exchange wherein the counterion of the peptide is exchanged with a pharmaceutically acceptable counterion, comprising:
a) loading a peptide onto a RP-HPLC column;
b) washing the column with an aqueous solution of a pharmaceutically acceptable counterion salt; and
c) eluting the peptide from the column with a solvent mixture of an organic solvent and an acid of the pharmaceutically acceptable counterion,
wherein the aqueous solution has a pH of at least 6 and the peptide is atosiban or lanreotide.

5. The peptide according to claim 4, wherein the pharmaceutically acceptable counterion salt is ammonium acetate, ammonium citrate, or ammonium pamoate.

6. The peptide according to claim 5, wherein the pH of the aqueous is 8.

7. The peptide according to claim 6, wherein the pH of the aqueous solution is adjusted by at least one base.

8. The peptide according to claim 7, wherein the base is ammonia, ammonium hydroxide, methylamine, ethylamine, dimethylamine, diethylamine, methylethylamine, trimethylamine or triethylamine.

9. The peptide according to claim 7, wherein the base is ammonium hydroxide.

10. The peptide according to claim 4, wherein the solvent mixture has a pH of less than 6.

11. The peptide according to claim 4, wherein the organic solvent is at least one of acetonitrile, methanol, ethanol, isopropanol or THF.

12. The peptide according to claim 11, wherein the organic solvent is acetonitrile.

13. The peptide according to claim 4, wherein the acid of the pharmaceutically acceptable counterion is acetic acid, citric acid or pamoic acid.

14. The peptide according to claim 4, wherein the eluted peptide has no more than 0.25% by weight of residual counterion.

15. The peptide according to claim 4, wherein the eluted peptide has no more than 200 parts per million of residual counterion.
